(19) Europäisches Patentamt
European Patent Office
Office européen des brevets

(11) Veröffentlichungsnummer: **0 535 459 A1**

# (12) EUROPÄISCHE PATENTANMELDUNG

(21) Anmeldenummer: **92115917.4**

(51) Int. Cl.5: **G03F 7/038**, G01N 33/543

(22) Anmeldetag: **17.09.92**

(30) Priorität: **30.09.91 DE 4132466**

(43) Veröffentlichungstag der Anmeldung:
**07.04.93 Patentblatt 93/14**

(84) Benannte Vertragsstaaten:
**BE CH DE DK ES FR GB IT LI NL SE**

(71) Anmelder: **BAYER AG**

**W-5090 Leverkusen 1 Bayerwerk(DE)**

(72) Erfinder: **Heiliger, Ludger, Dr.**
**Carl-Rumpff-Strasse 8**
**W-5090 Leverkusen 1(DE)**
Erfinder: **Kuckert, Eberhard, Dr.**
**Am Scherfenbrand 67**
**W-5090 Leverkusen 1(DE)**

(54) **Photochemisch verknüpfbare Polymere.**

(57) Photochemisch verknüpfbare Polymere der Formel (I)

$P\text{-}(A)_n$ (I)

in welcher
P eine polymere Komponente,
A ein photochemisch reaktiver Teil und
n die Zahl 1 oder 2 bedeutet,
sowie deren Verwendung für immunologische Zwecke.

EP 0 535 459 A1

Die vorliegende Erfindung betrifft neuartige, photochemisch verknüpfbare Polymere, Verfahren zu deren Herstellung sowie ihre Verwendung zur photochemischen Immobilisierung und/oder Markierung von biologisch aktiven Molekülen, wie beispielsweise Proteinen oder Nukleinsäuren.

In der WO 89/053 29 werden Polymeroberflächen von fertigen, an sich wasserunlöslichen Polymeren, wie Mikrotitrierplättchen aus Polystyrol oder Polymethylmethacrylat mittels photochemisch aktiven, biotinylierten Verbindungen in wässriger Lösung anhand polymeranaloger Photoreaktion modifiziert. Die photochemische Verknüpfung der photochemisch reaktiven monomeren Verbindung mit dem Polymeren geschieht in heterogener Phase mit dem von der Lösung benetzten Teil des Polymeren, d.h. nur an den sich berührenden Oberflächen der beiden Phasen. Die hohe Oberflächenspannung des Wassers, die sich in der schlechten Benetzbarkeit des Kunststoffplättchens niederschlägt, sorgt zudem für einen extrem schlechten Kontakt beider Komponenten, die zur Reaktion gelangen sollen. Daraus ergibt sich eine schlechte Quantenausbeute der Photoverknüpfungsreaktion und eine lange Bestrahlungszeit (1,5 h), um überhaupt Verknüpfungsreaktionen erfolgreich durchführen zu können. Die so modifizierte Polymeroberfläche läßt sich prinzipiell nur noch für die Immobilisierung von Biomolekülen über die an sich bekannte mehrstufige Verknüpfung von Titrierplättchen - Biotin - Avidin - (oder Streptavidin -) Biotin - Biomolekül Sequenz erreichen. Dazu ist es notwendig, das zu immobilisierende Biomolekül zuerst einer Funktionalisierung mit Biotin zu unterwerfen, um die Immobilisierung überhaupt erst zu ermöglichen. Dabei ist die eigentliche Immobilisierung eines biotinylierten Biomoleküls nur in einem extrem engen Konzentrationsbereich des photochemischen Verknüpfungsreagenz experimentell zugänglich, weil (Strept-) Avidin, als mehrfachfunktionelles Reagenz gegenüber Biotin, gleichermaßen als Brückenglied in der Verknüpfungssequenz, wie auch als Blockiersubstanz (d.h. Vernetzung zwischen den einzelnen Polymermolekülen der Titrierplättchen) reagieren kann. Das bedeutet, daß bei niedriger Konzentration an Biotin-Ankergruppen eine Verknüpfung mit (biotinylierten) Biomolekülen über (Strept-) Avidin nur in geringen Ausbeuten verlaufen kann, während bei hohen Konzentrationen von Biotin-Ankergruppen eine Totalvernetzung mit dem Mikrotitrierplättchen und damit keine Verknüpfung mit (biotonylierten) Biomolekülen mehr erfolgt.

Erfindungsgemäße Polymere lassen sich dagegen in homogener Phase in einem Reaktionsschritt mit nativen Biomolekülen, wie Nukleinsäuren oder Proteinen photochemisch ohne Modifizierung oder Funktionalisierung direkt verknüpfen und somit nicht nur für eine direkte, spezifische Immobilisierung, sondern darüberhinaus auch über die photochemische Markierung zur Quantifizierung von Biomolekülen einsetzen. Durch den gezielten Einbau des photochemisch reaktiven Teils, tritt auch bei hohem Umsatz der Verknpüfungsreaktion keine Vernetzung auf.

Es wurden nun photochemisch verknüpfbare Polymere der allgemeinen Formel (I) gefunden

$P\text{-}(A)_n$ (I)

in welcher

- P eine polymere Komponente,
- A ein photochemisch reaktiver Teil und
- n die Zahl 1 oder 2 bedeutet.

Die polymere Komponente P kann linear, verzweigt oder vernetzt sein und läßt sich beispielsweise durch radikalische Polymerisation oder Polykondensation herstellen. Polyurethane und Polyharnstoffe sind ebenfalls geeignet.

Als Monomerbausteine der polymeren Komponente kommen beispielsweise Acryl-, Methacryl-, Vinyl- oder Styryl-Reste oder Mischungen davon in Frage. Dabei kann es sich beispielsweise um deren Säure-, Ester-, Amid- oder Keton-Derivate handeln. Die Monomerbausteine können reaktive oder aktivierbare Gruppen enthalten, welche die kovalente Bindung beispielsweise zu einem Chelatbildner ermöglichen. Derartige Gruppen können beispielsweise Säurehalogenid-, Imidester-, Benztriazolyl-, Isocyanato-, Isothiocyanato-, Oxiran oder Diimid-Gruppen sein. Bevorzugte Monomerbausteine sind (Meth)-Acrylsäurechlorid, (Meth)Acrylsäure-N-hydroxy-succinimidester, (Meth)Acrylsäure-N-hydroxy-phthalimidester, N-(Meth)-acryloylbenztriazol, 3- oder 4-Isothiocyanatophenyl-(meth)acrylat, 2-Isocyanatoethyl(meth)-acrylat, Isocyanatoisopropenylbenzol, Isopropenyl-$\alpha,\alpha$-dimethylbenzylisocyanat, Vinyloxiran oder eine Kombination aus (Meth)Acrylsäure mit Carbodiimiden.

Als Chelatbildner im Sinne der Erfindung werden alle Strukturen verstanden, die in der Lage sind, ein-, zwei- und dreiwertige Metallionen reversibel zu komplexieren.

Mögliche Chelatbildner sind z.B. die durch Einsatz von

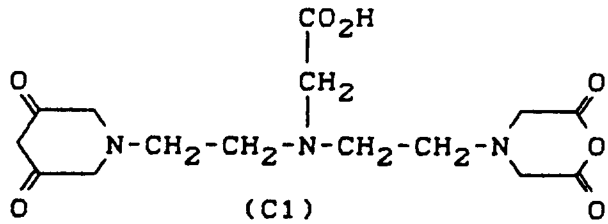

(C1)

oder

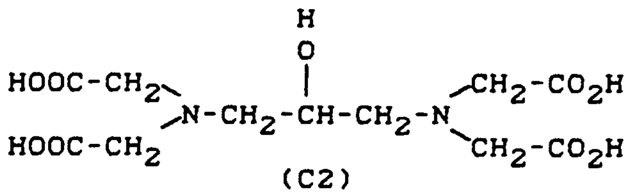

(C2)

gewinnbaren (z.T. nach Öffnung cyclischer Säureanhydridfunktionen) sowie die Verbindungen der Formel

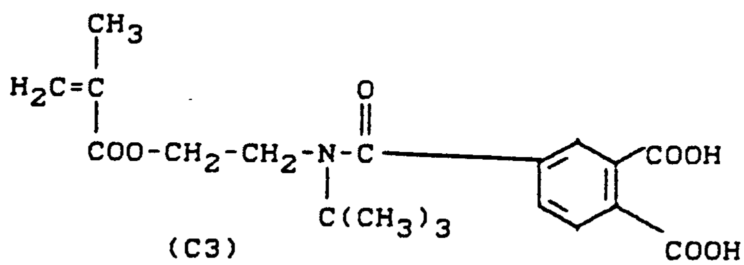

(C3)

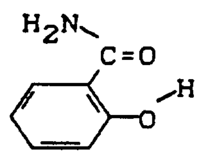

Salicylsäureamid

(C4)

Eine Vielzahl von geeigneten Chelatbildnern sind beschrieben bei L. Yuanfang und W. Chuanchu, Pure and Applied Chemistry, Vol. 63 No. 3, 427-463 (1991).

Die Verbindung C3 erhält man durch Umsetzung von Benzoltricarbonsäureanhydridchlorid mit n-tert.-Butyl-2-aminoethylmethacrylat in einem organischen Lösungsmittel, wie beispielsweise Dioxan, zwischen Temperaturen von -30 und 110 °C gegebenenfalls in Gegenwart einer Base, wie beispielsweise Triethylamin oder Pyridin, und anschließender Verseifung der Anhydridfunktion.

Als Monomerbausteine sind Verbindungen der Formel (II) geeignet

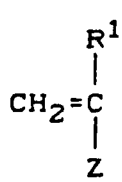

(II)

in welcher

Z für Wasserstoff, $C_1$-$C_{20}$-Alkyl, vorzugsweise $C_1$-$C_{15}$-Alkyl, insbesondere $C_1$-$C_6$-

Alkyl, $-CO-OR^2$, $-CO-NR^3R^4$ oder $-OOCR^5$ steht und

$R^1$, $R^2$, $R^3$, $R^4$ und $R^5$ unabhängig voneinander Wasserstoff oder $C_1$-$C_{20}$-Alkyl, vorzugsweise $C_1$-$C_{15}$-Alkyl, insbesondere $C_1$-$C_6$-Alkyl, bedeuten.

Als weitere monomere Bausteine kommen beispielsweise Styrol, α-Methylstyrol oder Verbindungen der Formel (III) in Frage

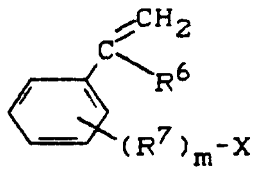

(III)

in der

- $R^6$ Wasserstoff oder Methyl,
- $R^7$ $CH_2$ oder $SO_2$,
- m Null oder 1 und
- X Halogen, $SO_2$-$CH_2$-$CH_2$-Halogen, OMe, $SO$-$CH_3$ oder Methyl

bedeuten.

Halogen steht dabei vorzugsweise für Chlor, Brom oder Iod, insbesondere Chlor oder Brom, und Me für ein Äquivalent eines Metalls, beispielsweise Natrium, Kalium, Casium oder Ammonium.

Ebenso kommen 1- und 2-Vinylnaphthalin, 1-Vinylcarbazol und Verbindungen in Frage, die zu solchen der Formel (III) analog sind, als aromatischen Grundkörper jedoch Naphthalin oder Carbazol enthalten, sowie sich von aromatischen Aminen, Phenolen, aromatischen Hydroxycarbon-, Hydroxysulfon-, Aminocarbon- und Aminosulfonsäuren ableitenden (Meth)Acrylamide und (Meth)Acrylate der Formel (IV)

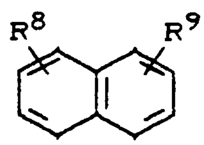

(IV)

in der

- $R^8$ für Wasserstoff, $SO_3H$, COOH, $SO_3Me$ oder COOMe steht, wobei Me ein Äquivalent eines Metalls wie beispielsweise Natrium ist, bedeutet und
- $R^9$

für 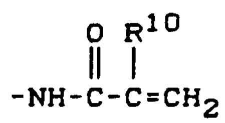 oder 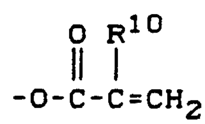

mit $R^{10}$ = Wasserstoff oder Methyl

steht.

Die Polykondensate, Polyurethane oder Polyharnstoffe sind bekannt, deren allgemeine Herstellungsverfahren werden in Houben-Weyl, Makromolekulare Chemie, Teil 1, (1987), S. 555-608 beschrieben. Zusätzlich sind im Teil 2, S. 1443-1457 und 1561-1751 eine große Anzahl dieser Polymere detailliert für verschiedene Anwendungszwecke näher beschrieben.

Die Monomerbausteine der Komponente P können wasserlöslich machende ionische oder nichtionische Gruppen enthalten. Vorzugsweise enthalten die Monomere ionische wasserlöslich machende Gruppen.

Es kann sich dabei beispielsweise um Acrylsäure, Methacrylsäure, Acrylamid, Methacrylamid oder Derivate davon handeln. Als Derivate kommen z.B. in Frage: 2-Acryloylamino-2-methyl-propansulfonsäure, Dialkylamino-alkyl-(meth)-acrylate und Dialkylamino-alkyl-(meth)-acrylamide wie Dimethylaminoethyl-methacrylat, Dimethylamino-propyl-acrylamid und die sich von solchen (Meth)acrylaten und (Meth)acrylamiden ableitenden quarternierten Verbindungen. Ferner kommen beispielsweise in Frage: N-Vinylpyrrolidon, N-Vinylpiperidon, N-Vinylcaprolactam, N-Vinylformamid, N-Vinylacetamid, N-Vinyl-N-methyl-acetamid und N-Vinyl-O-methylurethan.

Die oben genannten Monomerbausteine können untereinander in Form von statistischen, alternierenden oder verzweigten Block-, Pfropf- oder Kammpolymeren kombiniert werden. Bevorzugt sind statische Copolymere sowie Homopolymere.

Bevorzugte Monomerbausteine enthalten wasserlöslichkeitsvermittelnde Gruppen, um so dem Polymer Wasserlöslichkeit zu vermitteln, die für die Photoverknüpfung mit biologisch aktiven Substraten vorteilhaft ist. Von den wasserlöslichkeitsvermittelnden Gruppen sind Carbon- und Sulfonsäure-Gruppen sowie deren konjugierte Basen besonders bevorzugt. Besonders bevorzugt sind polymere Komponenten P, die für ein-, zwei und drei-wertige Kationen, wie beispielsweise Lanthaniden, insbesondere Europium, chelatisierend wirken.

Nichtvernetzte, nichtionische Polymere P haben mittlere Molekulargewichte ($\overline{M}_w$) zwischen 1 000 und 10 000 000, bevorzugt zwischen 5 000 und 2 000 000. Polykondensationsprodukte haben vorzugsweise mittlere Molekulargewichte zwischen 5 000 und 100 000.

Ionische Polymere haben in der Regel eine Grenzviskosität von mindestens 0,1 dl/g, bevorzugt 0,5 bis 20, insbesondere 1-10 dl/g gemessen in 0,9 % iger wäßriger NaCl-Lösung bei 20 °C.

Als photochemisch aktive Bausteine A kommen beispielsweise Acridin-Farbstoffe, Furocumarine, Phenanthridine, Phenazine, Phenothiazine, Chinoline, Antracycline, Netropsin, Distamycin oder bis-Benzimidazole in Frage, vgl. die folgende Aufstellung (Tabelle 1).

Tabelle 1

Aufstellung von fotochemisch aktiven Teilen A

| Baustein A | Literaturzitat |
| --- | --- |
| - Acridin Farbstoffe: | Lerman, J. Mol. Biol. 3:18 (1961); Bloomfield et al, "Physical Chemistry of Nucleic Acids", Chapter 7, pp. 429-476, Harper and Rowe, NY (1974) |
| Proflavin, Acridin Orange, Chinacridin | Miller et al, Biopolymers 19:2091 (1980) |
| - Phenanthridine: | Bloomfield et al, s.o. Miller et al, s.o. |
| Ethidium, Coralin | Wilson et al, J. Med. Chem. 19:1261 (1976) |
| Ellipticin, Ellipticin-Kation und Derivate | Fety et al, FEBS Letters 17.321 (1971); Kohn et al, Cancer REs. 35:71 (1976); LePecq et al, PNAS (USA) 71:5078 (1974); Pelaprat et al, J. Med. Chem. 23:1330 (1980) |
| - Phenazine: 5-Methylphenazin-Kation | Bloomfield et al, s.o. |
| - Phenothiazine: Chlorpromazin | Bloomfield et al, s.o. |
| - Chinoline: Chlorchinon, Chinin | Bloomfield et al, s.o. |
| - Antracycline: B-Rhodomycin A Daunamycin | Bloomfield et al, s.o. |

| Baustein A | Literaturzitat |
|---|---|
| - Furocumarine: | |
| Angelicin | Venema et al, MGG, Mol. Gen. Genet. 179; 1 (1980) |
| 4,5'-Dimethylangelicin | Vedaldi et al, Chem. Biol. Interact. 36: 275 (1981) |
| Psoralen | Marciani et al, Z. Naturforsch B 27 (2): 196 (1972) |
| 8-Methoxypsoralen | Belognzov et al, Mutat. Res. 84:11 (1981); Scott et al, Photochem. Photobiol. 34:63 (1981) |
| 5-Aminomethyl-8-methoxypsoralen | Hansen et al, Tet. Lett. 22; 1847 (1981) |
| 4,5,8-Trimethylpsoralen | Ben-Hur et al. Biochem. Biophys. Acta 331:181 (1973) |
| 4'-Aminomethyl-4,5,8-trimethylpsoralen | Issacs et al, Biochem. 16:1058 (1977) |
| Xanthotoxin | Hradecma et al, Acta Virol. (Engl. Ed.) 26:305 (1982) |
| Khellin | Beaumont et al, Biochem. Biophys. Acta 608:1829 (1980) |

Furocumarine und Phenanthridine sind als fotochemisch reaktiver Teil A bevorzugt.
Besonders bevorzugt sind Angelicine der allgemeinen Formel (V)

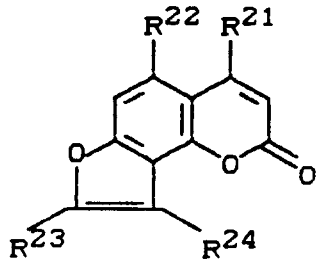

(V)

in welcher

$R^{21}$, $R^{22}$ und $R^{23}$ unabhängig voneinander Wasserstoff oder $C_1$-$C_4$-Alkyl bedeuten und

$R^{24}$ Wasserstoff, $C_1$-$C_4$-Alkyl, durch Hydroxy, $C_1$-$C_4$-Alkoxy, Amino, Halogen und/oder den Rest

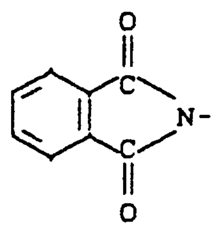

substituiertes $C_1$-$C_4$-Alkyl bedeutet,
und Psoralene der allgemeinen Formel (VI)

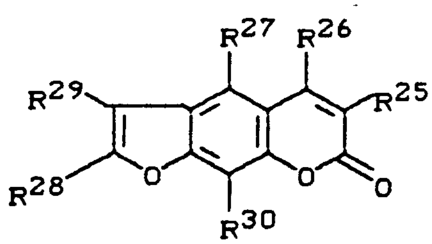

(VI)

in welcher

$R^{25}$, $R^{26}$, $R^{27}$ und $R^{28}$ unabhängig voneinander Wasserstoff oder $C_1$-$C_4$-Alkyl bedeuten und
$R^{29}$ Wasserstoff, $C_1$-$C_4$-Alkyl, durch Hydroxy, $C_1$-$C_4$-Alkoxy, Amino, Halogen und/oder

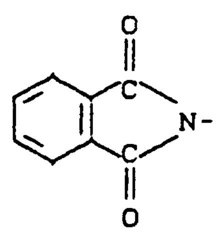

substituiertes $C_1$-$C_4$-Alkyl bedeutet und
$R^{30}$ Wasserstoff, Hydroxy, $C_1$-$C_4$-Alkyl, Carboxy, $C_1$-$C_4$-Alkoycarbonyl oder $C_1$-$C_4$-Alkoxy bedeutet.

In den Formeln (V) und (VI) stehen $R^{21}$, $R^{22}$, $R^{23}$ und $R^{25}$, $R^{26}$, $R^{27}$ und $R^{28}$ unabhängig voneinander vorzugsweise für Wasserstoff oder Methyl.

In Formel V steht $R^{24}$ vorzugsweise für Wasserstoff, Methyl oder Aminomethyl.

In Formel (VI) stehen vorzugsweise $R^{29}$ für Wasserstoff, Methyl, Hydroxymethyl oder Aminomethyl und $R^{30}$ für Wasserstoff, Methyl, Hydroxy, Carboxy oder Methoxycarbonyl.

Besonders bevorzugt sind 4'-Aminomethyl-4,5'-dimethylangelicine der Formel (Va)

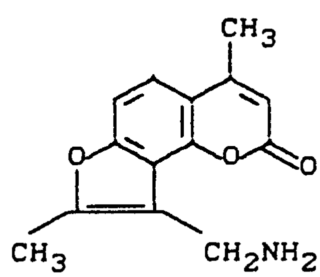

(Va)

und die Verbindung der Formel (VIa)

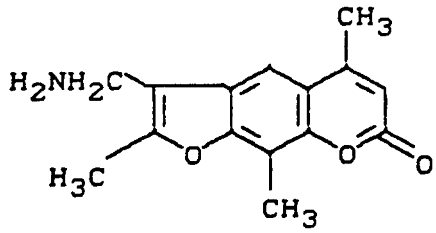

(VIa)

Beispielhaft seien die folgenden Angelicine der Formel (V) genannt:

| $R^1$ | $R^2$ | $R^3$ | $R^4$ |
|---|---|---|---|
| H | H | H | H |
| $CH_3$ | H | $CH_3$ | H |
| $CH_3$ | $CH_3$ | $CH_3$ | $CH_2OH$ |
| $CH_3$ | H | $CH_3$ | $CH_2OCH_3$ |
| $CH_3$ | H | $CH_3$ | $CH_2NH_2$ |
| $CH_3$ | H | $CH_3$ | $CH_2Cl$ |
| $CH_3$ | H | CH | 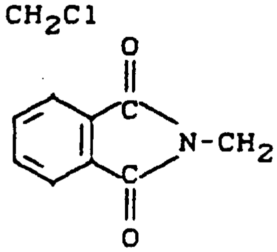 |

Im einzelnen seien beispielsweise die folgenden erfindungsgemäßen Polymere der Formel (I) genannt, wobei unter Angelicin A 4'-Aminomethyl-4,5'-dimethylangelicin zu verstehen ist:

Angelicin A-[Polystyrolbeads]-Angelicin A

Angelicin A-(Polymer 1)

Angelicin A-(Polymer 2)-Angelicin A

Angelicin A-(Polymer 3)-Angelicin A

Monomereinheit des Polymer 1 enthaltend die chelatbildende Gruppe:

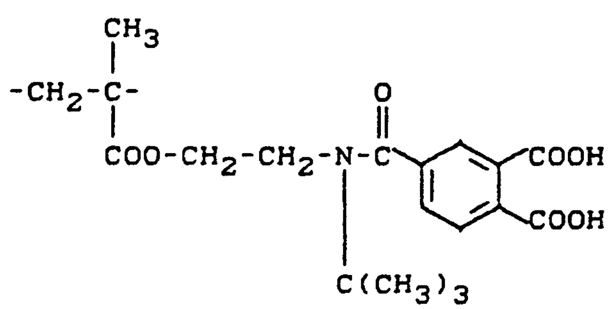

Monomereinheit des Polymer 2:

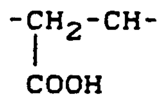

Momomereinheit des Polymer 3, enthaltend die chelatbildende Gruppe:

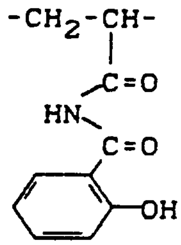

Unter Polystyrolbead versteht man mikroskopisch kleine (d.h. ca. 10-1000 $\mu$) makroporöse Polystyrolkügelchen.

Zusätzlich können erfindungsgemäße photochemisch verknüpfbare Polymere noch durch die Herstellungsart bedingt Radikalkettenstarter-Reste enthalten, welche den Resten B-L in Formel (I) entsprechen.

Die Bausteine A und P der erfindungsgemäßen Polymere sind beispielsweise über eine Ester-, Sulfonsäureester-, Amid-, Sulfonamid-, Urethan-, Thiourethan-, Harnstoff-, Thio-Harnstoff-, Ether-, Amin- und Sulfid-Gruppe miteinander verknüpft. Falls eine erhöhte Beweglichkeit der Bausteine A und/oder P erwünscht ist, können zwischen den Verknüpfungsgruppen Reste wie beispielsweise $C_1$-$C_{20}$-Alkylen, vorzugsweise $C_3$-$C_{15}$-Alkylen, insbesondere $C_5$-$C_{10}$-Alkylen, $C_6$-$C_{10}$-Arylen-$C_2$-$C_{10}$-Alkylen, vorzugsweise Phenylen- bzw. Naphthylen-$C_2$-$C_8$-Alkylen, oder $(CH_2\text{-}CH_2\text{-}O)_1$ mit 1 = 1-20, vorzugsweise 3-15, insbesondere 5-10, eingebaut sein.

Bevorzugt sind die Bausteine A und P über eine Amid-, Harnstoff-, Ester- oder Urethan-Gruppe verknüpft, besonders bevorzugt sind Amid- und Harnstoff-Gruppen.

Die Monomerbausteine sind allgemein bekannt. Die polymere Komponente P läßt sich nach allgemein bekannten Polymerisationsverfahren herstellen.

So werden beispielsweise Vinylmonomere nach den üblichen Bedingungen der radikalischen Polymerisation mit Radikalkettenstartern, die den photochemisch reaktiven Teil A enthalten (co-) polymerisiert, d.h. bei Temperaturen von 30 bis 150 °C, wenn erwünscht oder bei festen Reaktanten in (inerten) Lösemittel, wie beispielsweise Dimethylacetamid, Dimethylformamid, Dimethylsulfoxid, (chlorierte) Aromaten, (chlorierte) Aliphaten, Ether, Ester, Ketone, Alkohole, bei atmosphärischem Druck oder bei gasförmigen Monomeren auch bei Überdruck in Autoklaven umsetzt. Wenn es gewünscht ist, können noch übliche Zusätze wie ($\overline{M}_w$-Regler wie beispielsweise Dodecanthiol oder n-Butanthiol, Vernetzer bei der Herstellung von Beads wie beispielsbeise Divinylbenzol, etc.) der Reaktionslösung beigesetzt werden.

Die den photochemisch reaktiven Teil A enthaltenden Radikalkettenstarter (Verbindungen der Formel (VII) s.u.) sind beispielsweise durch Umsetzung von amino- oder hydroxy-funktionellen Verbindungen, die den photochemisch reaktiven Teil A enthalten, mit (sulfon)säurechlorid- oder isocyanat-funktionellen Radikalkettenstartern zugänglich.

Der photochemisch reaktive Teil A, der in den Polymeren der Formel (I) enthalten ist, wird mit Hilfe von neuen photochemisch reaktiven Initiatoren der allgemeinen Formel (VII)

$A\text{-}L\text{-}B\{L\text{-}A\}_y$ (VII)

worin

- A der oben definierte photochemisch reaktive Teil ist,
- B ein radikalbildender Teil und
- L eine Linkergruppierung und
- y die Zahl 0 oder 1 bedeutet,

erhalten.

Als Linkergruppierung L kommen in Frage:
Sulfonsäureester, Ester, Sulfonamid, Amid, Urethan, Thiourethan, Harnstoff, Thio-Harnstoff, Ether, Amin, Sulfid.

Bevorzugte Linkergruppierungen sind Amide und Harnstoffe sowie Ester und Urethane. Amide und Harnstoffe sind besonders bevorzugt.

Die Linkergruppierung L verknüpft den fotochemisch reaktiven Teil und den radikalbildenden Teil B kovalent miteinander.

Falls eine erhöhte Beweglichkeit der Bausteine A und/oder B erwünscht ist, kann durch L auch eine Abstandshalterfunktion ausgeübt werden, wobei L dann aus folgenden Untereinheiten der Formel (VIII) bestehen kann:

$L^1$-R-$L^1$ (VIII),

worin

$L^1$ die bei L angegebenen Bedeutungen hat und

R für $C_1$-$C_{20}$-Alkylen, vorzugsweise $C_3$-$C_{15}$-Alkylen, besonders bevorzugt für $C_5$-$C_{10}$-Alkylen, für $C_6$-$C_{10}$-Arylen-$C_2$-$C_{10}$-Alkylen, vorzugsweise für Phenylen- bzw. Naphthylen-$C_2$-$C_8$-Alkylen, für $(CH_2$-$CH_2$-$O)_n$ steht, worin

n 1 bis 20, vorzugsweise 3 bis 15 und besonders 5 bis 10, bedeutet.

Als radikalbildender Teil B kommen folgende Verbindungen in Frage:

1) Azostrukturen der allgemeinen Formel (IX)

$R^{11}$-N=N-$R^{12}$ (IX)

worin

$R^{11}$ und $R^{12}$ $C_1$-$C_{20}$-Alkyl, $C_3$-$C_{10}$-Cycloalkyl, $C_7$-$C_{20}$-Aralkyl oder die Gruppe

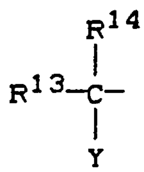

bedeuten, und

Y CN, $N_3$ oder

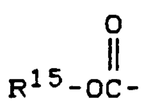, 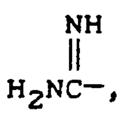, 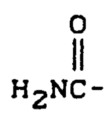, 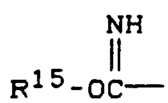

$R^{15}$-O-,

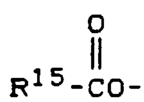,

$R^{15}$S-, NCS-, SCN-,

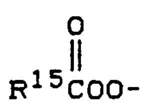,

HO-,

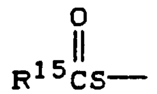 ,

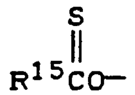 , 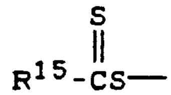 , 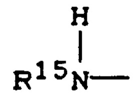

oder

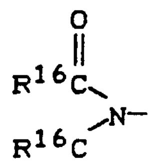

bedeutet,

$R^{13}$, $R^{14}$ und $R^{15}$ unabhängig voneinander $C_1$-$C_{20}$-Alkyl, $C_3$-$C_6$-Cycloalkyl bedeuten oder wenn $R^{13}$ und $R^{14}$ verknüpft sind $C_2$-$C_{30}$-Alkylen bedeuten oder zusätzlich einer der Reste $R^{13}$ oder $R^{14}$, jedoch nicht beide gleichzeitig, Phenyl, Tolyl, Xylyl, Benzyl oder Phenethyl bedeutet,

$R^{16}$ unabhängig $C_1$-$C_6$-Alkyl, $C_3$-$C_6$-Cycloalkyl oder $C_6$-$C_{12}$-Aryl bedeutet;

2) Tetraaryl/alkylethane der allgemeinen Formel (X)

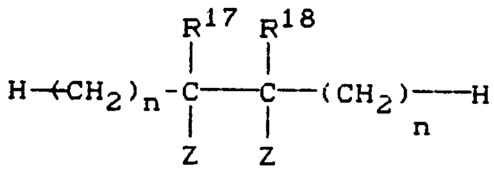 (X)

in welcher

Z für Hydroxy, $C_1$-$C_6$-Alkyl oder $C_6$-$C_{20}$-Aryl, insbesondere für Hydroxy, Methyl, Ethyl, n- oder iso-Propyl, Phenyl oder Naphthyl steht,

$R^{17}$ und $R^{18}$ unabhängig voneinander für $C_1$-$C_6$-Alkyl oder $C_6$-$C_{20}$-Aryl, insbesondere für Methyl, Ethyl, n- oder iso-Propyl, Phenyl oder Naphthyl stehen und

n für die Zahlen 1 bis 6, vorzugsweise 1, 2, 3, 4 oder 5, steht;

3) Dinitrile der Formel (XI)

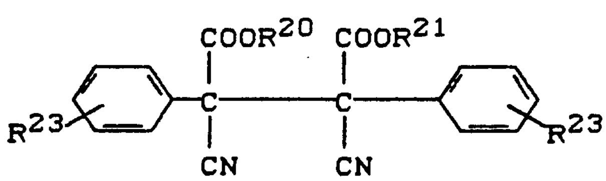 (XI)

in welcher

$R^{20}$, $R^{21}$ und $R^{23}$ unabhängig voneinander für oder $(CH_2)_m$-H stehen,

worin

m für die Zahlen 1 bis 6, vorzugsweise 1, 2, 3, 4 oder 5 steht;

4) Peroxide der allgemeinen Formel (XII)

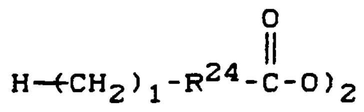

(XII)

in welcher

l die Zahlen 0 bis 6, vorzugsweise 0, 1, 2, 3 oder 4, insbesondere 0, 1 oder 2, bedeutet und

$R^{24}$ Phenylen, Naphthylen, $C_3$-$C_6$-Alkylen oder $C_3$-$C_6$-Cycloalkylen bedeutet.

Die unter 1 und 4) genannten Strukturen der Bausteine B sind bevorzugt.

Die Verbindungen der Formeln (IX) bis (XII) sind allgemein bekannt (vgl. beispielsweise US-PS 3 956 269, Houben-Weyl, Makromolekulare Stoffe, Teil 1, S. 16-19).

Bevorzugte Azostrukturen der Formel (IX) sind Verbindungen der Formel (IXa)

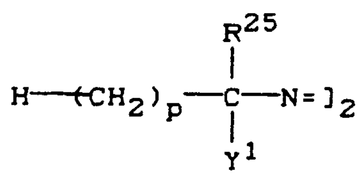

(IXa)

in welcher

p die Zahlen 1 bis 20, vorzugsweise 1 bis 15, insbesondere 2 bis 10, bedeutet,

$Y^1$ für CN, $N_3$, $COOR^{26}$ und

$R^{25}$ und $R^{26}$ unabhängig voneinander $C_1$-$C_6$-Alkyl, insbesondere Methyl, Ethyl, n- oder iso-Propyl, oder $C_3$-$C_6$-Cycloalkyl, insbesondere Cyclopropyl, Cyclopentyl oder Cyclohexyl, bedeuten.

Eine besonders bevorzugte Struktur der unter 1) genannten Bausteine B hat die Formel (IXb)

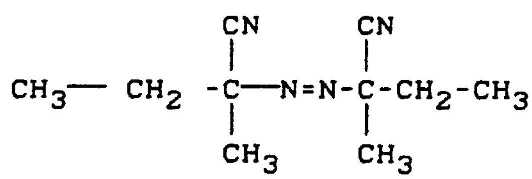

(IXb)

d.h., p bedeutet 2, $R^{25}$ $CH_3$ und $Y^1$ CN.

Eine besonders bevorzugte Struktur der unter 4) genannten Bausteine B entspricht der Formel (XI) mit 1 = 0 und $R^{24}$ = Phenylen.

Die Strukturen der Formeln (IX) bis (XII), die den radikalbildenden Teil ausmachen, tragen 1 (y in Formel (I) = 0) oder 2 (y ind Formel (I) = 2) reaktionsfähige Gruppen $X^1$ (vgl. Beschreibung der Formel (XIII).

In den Azostrukturen der Formel (IX) tragen die Reste $R^{11}$ und $R^{12}$ diese Gruppe(n). In den Strukturen der Formel (IXa) (IXb), (X) und (XII) sind die endständigen Wasserstoffatome durch diese Gruppe(n) ersetzt. Die Dinitrile der Formel (XI) tragen diese Gruppe(n) symmetrisch um die zentrale Bindung in $R^{20}$, $R^{21}$ und/oder $R^{23}$.

Im einzelnen seien beispielsweise die folgenden fotochemisch aktiven Initiatoren der Formel (VII) aufgezeigt, wobei unter Angelicin A das 4'-Aminomethyl-4,5'-dimethyl-angelicin zu verstehen ist.

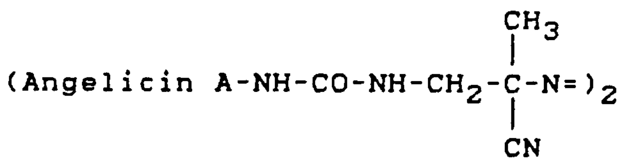

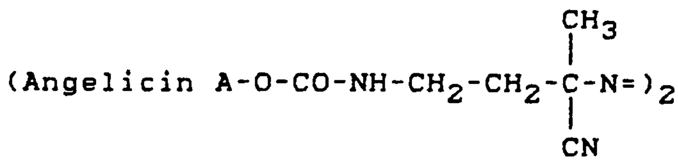

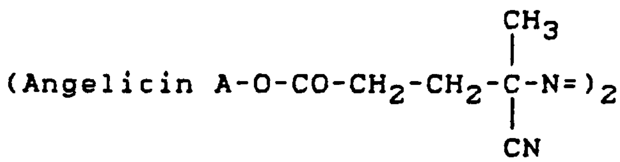

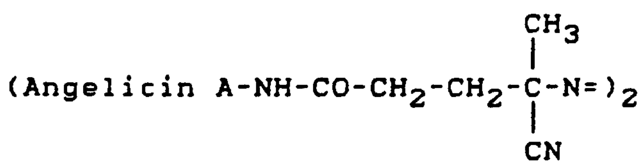

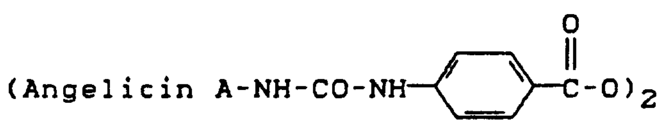

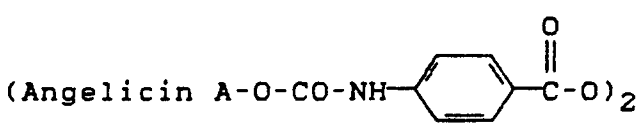

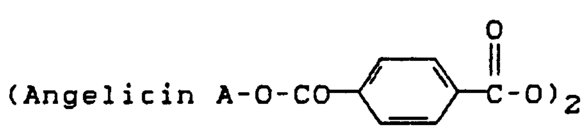

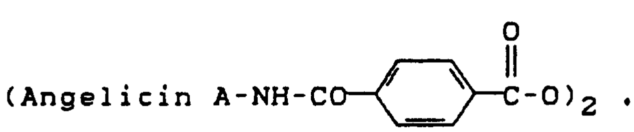

Zur Herstellung von fotochemisch aktiven Radikalketteninitiatoren der allgemeinen Formel (VII)

$$A\text{-}L\text{-}B\text{—}\left[\text{—}L\text{—}A\right]_y \qquad (VII)$$

werden radikalbildende Verbindungen der allgemeinen Formel (XIII)

$X^1$-B-($X^1$)$_y$ (XIII)

wobei

- B wie oben definiert ist und
- $X^1$ NCO, NCS, COCl, $CONHSO_2Cl$, COOH, CO-O-NHS, OH, $NH_2$, SH, Cl, Br oder I sein kann und
- y 0 oder 1, bevorzugt 0 ist,

mit 1 oder 2 Äquivalenten fotochemisch aktiven Substanzen A in gegenüber den unter $X^1$ genannten Gruppierungen chemisch inerten Lösungsmitteln, wie beispielsweise Chloraliphaten, Ketone, Nitrile, Sulfoxide, Sulfone u.ä., bei Temperaturen zwischen 0 und 40 ° C umsetzt.

Bei Verbindungen der allgemeinen Formel (XIII)

$X^1$-B-($X^1$)$_y$ (XIII)

in denen

- $X^1$ COCl oder $CONHSO_2Cl$ bedeutet,

ist dem Reaktionsgemisch zweckmäßigerweise ein Protonenfänger wie beispielsweise Pyridin oder Triethylenamin zuzusetzen, wohingegen, wenn X = COOH, die Umsetzung in Gegenwart von Carbodiimiden, beispielsweise Dicyclohexylcarbodimid, durchgeführt wird.

Als Verbindung der Formel $X^1$-B-($X^1$)$_y$ werden in das erfindungsgemäße Verfahren bevorzugt Verbindungen, in denen $X^1$ = NCO und COCl bedeutet, eingesetzt.

Die Reaktion erfolgt bevorzugt zwischen 0 und 30 ° C, besonders bevorzugt zwischen 15 und 25 ° C und insbesondere bei ca. 20 ° C. Als Lösungsmittel werden bevorzugt $CH_2Cl_2$, Aceton oder Acetontril eingesetzt.

Die Verbindugnen der Formel (XIII) sind allgemein bekannt oder lassen sich nach allgemein bekannten Verfahren herstellen (vgl. z.B. US-PS 4 155 937).

Die Linkergruppierung L wird gebildet durch die Umsetzung von $X^1$ aus der Formel $X^1$-B-($X^1$)$_y$ mit der reaktiven Gruppe im fotochemisch reaktiven Baustein A, beispielsweise der Aminogruppe in Angelicin A.

Die Isolierung der fotochemisch aktiven Radikalkettenstarter erfolgt nach an sich bekannten Methoden, z.B. nach dem Abfiltrieren von gegebenenfalls gebildeten (ionischen) Nebenprodukten durch Abdampfen des Lösungsmittels im Hochvakuum, wenn niedrigsiedende Lösungsmittel verwendet werden, oder durch Ausfällung durch Zusatz eines geeigneten Fällungsmittels, wobei das erfindungsgemäße Produkt in der Regel rein anfällt. In den Fällen, in denen die Nebenprodukte nicht flüchtig oder nicht durch Umlösen oder Filtrieren von den erfindungsgmeäßen Verbindungen abtrennbar sind, erfolgt die Isolierung der erfindungsgemäßen Verbindungen durch an sich bekannte Methoden der Flüssigchromatographie, beispielsweise Säulenchromatographie oder präparative HPLC.

Durch den (thermisch) initiierten Zerfall des Radikalkettenstarters werden freie Radikale, die den photochemisch reaktiven Teil A enthalten, gebildet, die mit den ethylenisch ungesättigten Verbindungen zu neuen freien Radikalen reagieren, welche weitere ethylenisch ungesättigte Monomere addieren, wobei der Abbruch der Wachstumsreaktion durch Kombination zweier wachsender Radikale, durch deren Disproportionierung, Wasserstoffabstraktion oder Kettentransfer (z.B. bei Verwendung von Reglern) erfolgt. Bei Abbruch durch erstere Reaktion entstehen erfindungsgemäße Polymere der Formel A-P-A, wohingegen bei letzteren Reaktionen erfindungsgemäße Polymere der Formel A-P entstehen. Bei mehreren, gleichzeitig auftretenden Abbruchreaktionen bilden sich Gemische der erfindungsgemäßen Polymere aus beiden Formeln. Die Molmassen der erfindungsgemäßen Polymere sind durch an sich bekannte Variationen der experimentellen Details, wie Initiator-, Monomer-, gegebenenfalls Reglerkonzentration, Temperatur und Lösemittel einstellbar.

Durch Verwendung eines mercaptogruppenhaltigen photochemisch reaktiven Teils, A-SH kann die radikalische Kettenpolymerisation auch unter Verwendung nichtfunktioneller, herkömmlicher Radikalkettenstarter, wie beispielsweise Azoisobutyronitril oder Dibenzoylperoxid initiiert werden, wobei der mercaptogruppen-enthaltende Teil A dann gleichzeitig als Kettenüberträger und Molgewichtsregler fungiert.

$$A\text{-}SH + (M)_n^{\cdot} \longrightarrow A\text{-}S\text{-}(M)_n + H^{\cdot} \xrightarrow{M} A\text{-}P + H\text{-}M^{\cdot}$$

Weitere Verfahren zur Herstellung erfindungsgemäßer Polymere A-P sind dadurch gekennzeichnet, daß man Monomere M, die für die anionische Polymerisation geeignet sind, unter den an sich bekannten Bedingungen der anionischen Polymerisation in Gegenwart von anionischen Initiatoren, die den photochemisch reaktiven Teil A enthalten, umsetzt, und die Reaktion mit den üblichen protonenaktiven Reagenzien, wie beispeilsweise Methanol nach Erreichen der gewünschten Molmasse abbricht. Die anionischen Initiatoren sind beispielsweise durch Reduktion (gegebenenfalls in situ) des photochemisch reaktiven Teils A mit Natrium- oder Lithium-Metall, in Lösemittel wie Tetrahydrofuran zugänglich.

$$A + Li \longrightarrow A^{\ominus}\ Li^{\oplus} \xrightarrow{pM} A\text{-}(M)_p^{\ominus}\ Li^{\oplus} \xrightarrow{MeOH} + A\text{-}P\ LiOMe$$

Alternativ können auch sonstige an sich bekannte mono- und bifunktionelle Initiatoren (AnI) für die anionische Polymerisation, verwendet werden, indem der Abbruch der Wachstumsreaktion nach Erreichen der gewünschten Molmasse mit funktionalisierbaren Reagenzien wie beispielsweise $I_2$ oder Br-CN erfolgen kann, woran dann die Verknüpfung mit hydroxy- oder aminofunktionellen Verbindungen, die den photochemisch reaktiven Teil A enthalten, in polymeranaloger Weise erfolgt:

$AnI\text{-}(M)_p^{\ominus} + Br\text{-}CN \rightarrow AnI\text{-}(M)_p\text{-}Br + CN^{\ominus}$
$AnI\text{-}(M)_p\text{-}Br + A\text{-}NH_2 \rightarrow P\text{-}A$

Als weiteres Verfahren zur Herstellung erfindungsgemäßer Farbstoffe eignet sich die (lebende) kationische Polymerisation dazu befähigter Monomere unter an sich bekannten Bedingungen, d.h. in Gegenwart kationischer Initiatoren (KatI) wobei die Wachstumsreaktion nach Erreichen der gewünschten Molmasse durch amino-, thio-, hydroxyfunktionelle, den photochemisch reaktiven Teil enthaltende Verbindungen abgebrochen wird.

$$KatI^{\oplus} + pM \longrightarrow KatI\text{-}(M)_p^{\oplus} \xrightarrow{A\text{-}OH} P\text{-}A + H^{\oplus}$$

Als Modifizierung der lebenden kationischen Polymerisation ist die Inifer-Methode zur Herstellung erfindungsgemäßer Polymerer geeignet, (vergl. Kennedy, J.P.: Polymer J. 12, 609, (1980), wo die Funktion des Initiators und Kettentransferagenz von ein und derselben Verbindung ausgeübt werden.

Die funktionellen Endgruppen können dann in polymeranaloger Weise mit amino- oder thio-funktionellem, photochemisch reaktivem Teil A enthaltender Verbindung reagiert werden.

Als weiteres Verfahren für die Herstellung erfindungsgemäßer Polymere A-P-A eignen sich Polykondensationsreaktionen, indem man nach an sich bekannten Verfahren zur Polykondensation befähigte Monomere, wie beispielsweise Diole oder Diamine und Bis-Isocyanate oder Bis-Säurechloride, einer Kondensationsreaktion unterwirft, deren Abbruch nach Erreichen der gewünschten Molmasse mit photochemisch reaktivem Teil A enthaltenden Verbindungen erfolgt.

Die durch diese Verfahren erhaltenen erfindungsgemäßen Polymere können, falls sie funktionelle Gruppierungen besitzen, in an sich bekannten polymeranalogen Reaktionen weiter umgesetzt werden, um so das gewünschte Eigenschaftsbild zu erreichen, z.B. Wasserlöslichkeit.

Zweckmäßiger und einfacher ist es, die gewünschten Eigenschaften durch die Polymeristion von geeigneten Monomeren einzustellen. Hier sind radikalisch polymerisierbare Monomere in Ihrer Vielseitigkeit der chemischen Eigenschaften den anderen Monomeren überlegen. Daher ist das Verfahren der freien radikalischen Kettenpolymerisation unter Verwendung von Radikalkettenstartern oder Kettenüberträgern, die den photochemisch reaktiven Teil A enthalten, bevorzugt.

Die erfindungsgemäßen photochemisch verknüpfbaren Polymere der Formel (I) lassen sich für immunologische Zwecke wie beispielsweise für Gen-Sonden-Tests oder zur Immobilisierung und Markierung von

biologischen Substanzen, wie beispielsweise DNA, verwenden.

Die nachfolgenden Beispiele dienen nur der Erläuterung und sind nicht so auszulegen, als ob sie die Art und den Umfang der vorliegenden Anmeldung in irgendeiner Weise einschränken.

## Beispiele

A) Herstellung von photochemisch verknüpfbaren Polymeren der Formel (I):

### Beispiel 1

Angelicin A-[Polystyrolbeads]-Angelicin A

Radikalische Suspensionspolymerisation von Styrolbeads mit Initiator gemäß Beispiel 5.

In einen Dreihalskolben, bestückt mit Hochgeschwindigkeitsblattrührer, Thermometer und Tropftrichter, wird folgende Mischung gegeben:
80 ml entionisiertes Wasser
9 g einer 10 %igen wäßrigen Mowiol 40-88®-Lösung (Hoechst AG) 0,09 g Natriumhydroxid.

Der Reaktionskolben wird mit Stickstoff gespült und der Inhalt auf 75 °C erhitzt und bei 750 UpM gerührt.

Innerhalb von 30 min wird ein Gemisch aus 30 g Styrol, 1,9 g Divinylbenzol, 5,6 g Cyclohexan und 3 g des Radikalstarters aus Beispiel 5 zudosiert und 2 h weitergerührt. Anschließend werden 5 ml entionisiertes Wasser und 4,5 g einer 10 %igen wäßrigen Mowiol 40-88®-Lösung zugegeben und noch 6 h weitergerührt.

Das Cyclohexan wird aus der Reaktionsmischung unter Wasserstrahlvakuum abdestilliert, der Ansatz nach dem Abkühlen mit 500 ml entionisiertem Wasser aufgefüllt, die Perlen sedimentieren lassen und durch ein 40 µm großes Sieb gegossen. Das so isolierte Produkt wird mit Methanol gewaschen und getrocknet.

Die Polystyrolbeads haben eine Teilchengröße von 200-300 µm.

### Beispiel 2

Angelicin A-(Polymer 2) Angelicin A

Radikalische Polymerisation von Acrylsäure mit Initiator gemäß Beispiel 5

Unter Stickstoff werden 5 g Acrylsäure in 10 ml Dimethylsulfoxid und 10 ml entionisiertes Wasser gelöst, auf 65 °C erhitzt und 50 mg des Radikalstarters aus Beispiel 5 zugegeben. Nach 2 h Rühren wird die hochviskose Lösung mit 20 ml entionisiertem Wasser verdünnt und über 15 h weiter gerührt. Das Rohpolymer wird in Aceton ausgefällt, abgesaugt, im Hochvakuum getrocknet. Die Grenzviskosität beträgt 8,5 dl/g in 0,9 %iger NaCl-Lösung bei 20 °C.

Beispiel 3

Angelicin A-(Polymer 1)-Angelicin A

Radikalische Polymerisation von

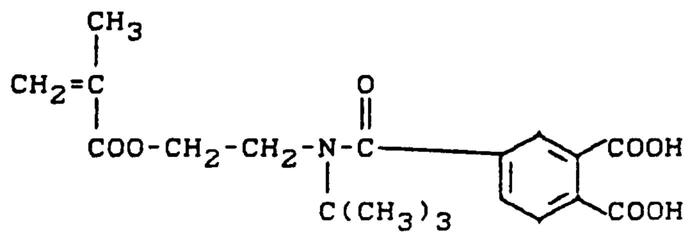

mit Initiator gemäß Bespiel 5

Unter den in Beispiel 2 beschriebenen Bedingungen werden 5 g des o.g. Monomeren mit 50 mg Radikalstarter gemäß Beispiel 5 unter Zugabe von 1,075 g Natriumhydroxid polymerisiert. Die Grenzviskosität beträgt 2,4 dl/g in 0,9 %iger NaCl-Lösung bei 20 ° C.

Beispiel 4

Angelicin A-(Polymer 3)-Angelicin A

Polymer 3:

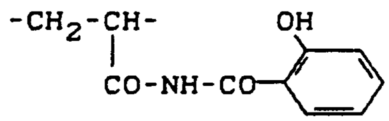

Unter Stickstoff werden 3 g des pulverisierten Polymers aus Beispiel 2 (Angelicin A-(Polymer 2)-Angelicin A) in einer Lösung aus 28,5 g (Salicylsäureamid in Xylol (50 ml) aufgeschläumt und destilliertes Thionylchlorid (24,8 g) tropfenweise zudosiert. Die Mischung wird 12 h bei 90 ° C reagieren lassen. Das resultierende Pulver wird abfiltriert, 3 x mit Xylol gewaschen und im Hochvakuum getrocknet.

Das Produkt zeigt zwei starke Absorptionsbanden im IR bei 1700 $cm^{-1}$ und 1660 $cm^{-1}$ charakteristisch für C = O, und eine mittelstarke Bande bei 3250 $cm^{-1}$ charakteristisch für N-H

B) Herstellung von fotochemisch aktiven Radikalkettenstartern der Formel (VII)

Beispiel 5

1 mmol 4'-Aminomethyl-4,5'-dimethylangelicin wird im 1 mmol 3,3'-Dicyano-3,3'-azodibutylisocyanat in 20 ml trockenem $CH_2Cl_2$ bei Raumtemperatur bis zum Verschwinden der IR-Bande der NCO-Gruppe unter Reinstickstoff gerührt, das Lösungsmittel im Rotationsverdampfer bei Raumtemperatur abgedampft. Der Rückstand ergibt das laut $^1$H-NMR reine erfindungsgemäße Produkt (angegeben ist die chemische Verschiebung $\delta$ in ppm),

| | |
|---|---|
| Harnstoffprotonen: | 5,4 und 5,82 ppm (Tripletts) |
| aromatische Protonen des Angelicin : | |
| | 7,25 ppm (Dublett), |
| | 7,32 ppm (Dublett), |
| | 6,07 ppm(Singulett) |

Beispiel 6

p,p'-Bisisocyanatomethylbenzoylperoxid wird wie unter Beispiel 5 beschrieben mit 4'-Aminomethyl-4,5'-dimethylangelicin in $CH_2Cl_2$ umgesetzt, isoliert und mittels IR-Spektroskopie charakterisiert

( 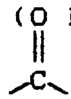 Harnstoff 1685 $cm^{-1}$).

Beispiel 7

1 mmol 4,4'-Dicyano-4,4'-azodipentanoylchlorid wird mit 1 mmol 4'-Hydroxymethyl-4,5'-dimethylangelicin in 20 ml trockenem $CH_2Cl_2$ und 1,1 mmol Pyridin bis zum Verschwinden der IR-Bande der COCl-Gruppe unter Reinstickstoff gerührt. Nach beendeter Reaktion wird vom Pyridiniumhydroxidchlorid abfiltriert, das Filtrat im Vakuum bei Raumtemperatur einrotiert. Man erhält ein laut $^1H$-NMR reines Produkt.
$^1H$-NMR:

| | | |
|---|---|---|
| Angelicin-Allylester-Protonen: | 4,4 ppm | |
| Angelicin aromatische Protonen: | 6,12 ppm | Singelett |
| | 7,3 ppm | Dubletts |
| | 7,37 ppm | |

C) Verwendung der Polymere der Formel (I) (Markierung von DNA)

Beispiel 8

Photoreaktion von Angelicin A-(Polymer 2)-Angelicin A (vgl. Beispiel 2) mit DNA

5 µl (0,4 µg DNA/µl) eines 1,7 kb-DNA-Fragments wurden mit 2 µl einer Lösung der polymeren, photoreaktiven Polyacrylsäure aus Beispiel 2 in Dimethylformamid (10.6 mg/ml) eine Stunde lang bei 365 nm belichtet. Zur Kontrolle des Bindungsverhaltens wurde das Reaktionsgemisch durch Agarosegelelektrophorese aufgetrennt. (0,5 % - 1 % Agarose, 1X TBE-Puffer (T B E = TRIS (Tris(hydroxymethoxy)-methylamin)-Borat-EDTA); 60 V; 2H). Die Reaktionsansätze, in denen eine Photoreaktion mit DNA durchgeführt wurde, zeigten ein deutliches "Nachschleppen" der DNA, die mit dem photochemisch modifizierten Polymer beladen war, verglichen zu unbelichteten Ansätzen von reiner 1,7 kb DNA.

Beispiel 9

Photoreaktion von Angelicin A-(Polymer 1)-Angelicin A (vgl. Beispiel 3) mit DNA

In der gleichen Weise wurde 2 µl einer Lösung des Polymeren aus Beispiel 3 in 0,1 M Natriumboratpuffer, pH 8,5 in einer Konzentration von 2 mg/ml mit 5 µl DNA-Lösung (1,7 kb; 0,4 µg/ul) umgesetzt. Die nachfolgende Elektrophorese ergab ebenfalls einen Shift in der Mobilität der markierten DNA, während die Kontrollexperimente mit unbelichteten Ansätzen und Startersubstanz nur unwesentliche Veränderungen im Laufverhalten zeigten.

**Patentansprüche**

**1.** Photochemisch verknüpfbare Polymere der Formel (I)

P-(A)$_n$ (I)

in welcher
- P eine polymere Komponente,
- A ein photochemisch reaktiver Teil und
- n die Zahl 1 oder 2 bedeutet.

**2.** Photochemisch verknüpfbare Polymere der Formel (I) gemäß Anspruch 1, worin A ausgewählt ist aus der Gruppe der Acridine, Furocumarine, Phenanthridine, Phenazine, Phenothiazine, Chinoline, Antracycline, Metropsin, Distamycin oder bis-Benzimidazole.

**3.** Photochemisch verknüpfbare Polymere der Formel (I) gemäß Anspruch 1, worin A für Angelicine der allgemeinen Formel (V)

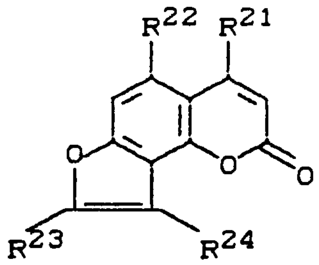

(V)

in welcher
- $R^{21}$, $R^{22}$ und $R^{23}$ unabhängig voneinander Wasserstoff oder $C_1$-$C_4$-Alkyl bedeuten und
- $R^{24}$ Wasserstoff, $C_1$-$C_4$-Alkyl, durch Hydroxy, $C_1$-$C_4$-Alkoxy, Amino, Halogen und/oder den Rest

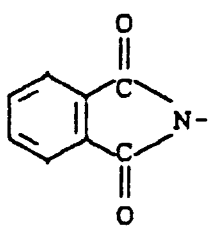

substituiertes $C_1$-$C_4$-Alkyl bedeutet,

und Psoralene der allgemeinen Formel (VI)

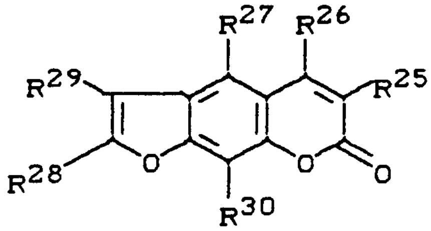

(VI)

in welcher
- $R^{25}$, $R^{26}$, $R^{27}$ und $R^{28}$ unabhängig voneinander Wasserstoff oder $C_1$-$C_4$-Alkyl bedeuten und
- $R^{29}$ Wasserstoff, $C_1$-$C_4$-Alkyl, durch Hydroxy, $C_1$-$C_4$-Alkoxy, Amino, Halogen und/oder

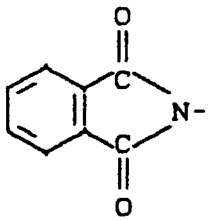

substituiertes $C_1$-$C_4$-Alkyl bedeutet und

$R^{30}$ Wasserstoff, Hydroxy, $C_1$-$C_4$-Alkyl, Carboxy, $C_1$-$C_4$-Alkoycarbonyl oder $C_1$-$C_4$-Alkoxy bedeutet,

steht.

4. Verwendung von photochemisch verknüpfbaren Polymeren der Formel (I) gemäß Anspruch 1 für immonologische Zwecke und zur Markierung von biologischen Substanzen.

Europäisches Patentamt

# EUROPÄISCHER RECHERCHENBERICHT

Nummer der Anmeldung

EP 92 11 5917

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | WO-A-8 905 329 (GLUETECH APS)<br>* Seite 10, Zeile 10 - Seite 15, Zeile 19 *<br>* Seite 17, Zeile 7 - Zeile 11 *<br>--- | 1-4 | G03F7/038<br>G01N33/543 |
| A | EP-A-0 187 332 (MOLECULAR DIAGNOSTICS)<br>----- | 1-4 | |

RECHERCHIERTE SACHGEBIETE (Int. Cl.5 )

C08F
G03F
G01N

Der vorliegende Recherchenbericht wurde für alle Patentansprüche erstellt

| Recherchenort | Abschlußdatum der Recherche | Prüfer |
|---|---|---|
| DEN HAAG | 17 DEZEMBER 1992 | ANDRIOLLO G.R. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)